# EUROPÄISCHE PATENTSCHRIFT

(11) **EP 2 111 805 B1**
(45) Veröffentlichungstag und Bekanntmachung des Hinweises auf die Patenterteilung: **12.12.2012**
(21) Anmeldenummer: 09003690.6
(22) Anmeldetag: 13.03.2009
(51) Int. Cl.: A61B 17/16, A61B 10/02, A61B 17/32

(54) **Knochenspanbohrer**
Bone graft harvesting drill
Dispositif de forage à copeaux d'os

(30) Priorität: 22.04.2008 DE 102008020178
(43) Veröffentlichungstag der Anmeldung: 28.10.2009
(73) Patentinhaber: Gebr. Brasseler GmbH & Co. KG, 32657 Lemgo (DE)
(72) Erfinder: Neumeyer, Stefan, 93458 Eschlkam (DE); Teller, Martin, 32657 Lemgo (DE); Küllmer, Michael, 32657 Lemgo (DE)
(74) Vertreter: Weber, Joachim

(56) Entgegenhaltungen:
- WO-A-98/48707
- WO-A-2008/149822
- DE-A1-102005 058 107
- DE-U1- 20 120 864
- US-A- 5 556 399
- US-A- 6 022 354

## Beschreibung

Die Erfindung bezieht sich auf einen Knochenspanbohrer und im Einzelnen auf einen Bohrer zur Gewinnung von Knochenspänen gemäß dem Oberbegriff des Anspruchs 1.

Bei der Implantatversorgung von Kiefern eines Patienten ist es im Rahmen des Bonemanagements erforderlich, Knochenspäne zu erzeugen. Diese sollen möglichst groß, d.h., in Form von groben Spänen vorliegen. Diese werden dann vom Zahnarzt oder Chirurgen an die aufzubauenden Bereiche gebracht, um dort Knochengewebe aufzubauen, an welchem wiederum Implantate verankert werden können.

Die DE 10 2005 058 107 A1 beschreibt ein chirurgisches Bearbeitungswerkzeug zum Herstellen einer Ausnehmung in einem Knochen. Das Werkzeug ist in Form einer Kegelschale oder Halbkugelschale ausgebildet und weist einen innenliegenden, dünnen Schaft auf. In der kegeligen oder halbkugelförmigen Schale sind spiralige Längsschlitze vorgesehen, die Schneidkanten bilden und durch welche das Knochenmaterial in den Innenraum der Schale gelangen kann. Dabei ist das Werkzeug so ausgebildet, dass kurze, kleine Späne erzeugt werden, welche durch die Schlitze leicht abtransportiert werden können.

Die DE 299 01 724 U1 beschreibt eine Vorrichtung zur Gewinnung von Knochenspänen, welche in Form eines rohrförmigen, hohlen Schneidwerkzeugs ausgebildet ist, an dessen hinterem Ende ein Behälter zur Aufnahme der Knochenspäne angeordnet ist.

Der Erfindung liegt die Aufgabe zugrunde, einen Knochenspanbohrer der eingangs genannten Art zu schaffen, welcher bei einfachem Aufbau und einfacher, kostengünstiger Herstellbarkeit zur Erzeugung von groben Knochenspänen besonders gut geeignet ist.

Erfindungsgemäß wird die Aufgabe durch die Merkmalskombination des Anspruchs 1 gelöst, die Unteransprüche zeigen weitere vorteilhafte Ausgestaltungen der Erfindung.

Erfindungsgemäß ist somit ein Knochenspanbohrer geschaffen, der einen relativ dicken Kopf aufweist. Dieser Kopf selbst ist nicht in üblicher Weise mit direkt schneidenden Schneiden versehen. Vielmehr ist der Kopf von seinem vorderen Endbereich zu seinem rückwärtigen Endbereich von zumindest einer Spanausnehmung durchdrungen. Diese verläuft bevorzugterweise diagonal und zur Schaftachse geneigt. Die Verschneidungskurve oder Schnittkante zwischen der Spanausnehmung und der Kontur des Kopfes erzeugt dabei eine Schneide, mit welcher grobe Knochenspäne abgeschnitten oder abgehobelt werden können. Diese Späne sind insbesondere auch sehr lang, so dass sie vom Operateur gut gegriffen und gehandhabt werden können. Die so erzeugten Späne eignen sich somit hervorragend für einen Wiederaufbau von Knochenbereichen. Besonders Vorteilhaft ist die durch den Mehrkant bedingte geringe Flächenauflage der Bohrerspitze. Dadurch wir die entstehende Reibungswärme reduziert und der Knochen nicht thermisch geschädigt. Ebenso wird das Schneidverhalten des Bohrers positiv beeinflusst, so dass ein mögliches Klemmen des Bohrers vermieden wird.

Erfindungsgemäß ist somit, im Gegensatz zum Stand der Technik, vorgesehen, dass lediglich eine einzige zylindrische Spanausnehmung den Kopf durchdringt. Dies führt zu einer präzisen und zuverlässigen Abfuhr relativ großer "abgehobelter" Späne, die besonders gut und wirkungsvoll zum Knochenaufbau verwendet werden können.

Demgegenüber sieht die Erfindung eine einzige Schneide vor, die von der einen, einzigen Spanausnehmung gebildet wird. Es liegt somit ein technisches Prinzip zugrunde, welches darin besteht, mittels einer einzigen Schneide durch eine maximal große Spanausnehmung möglichst große, durch den Operateur einzeln greifbare Späne zu gewinnen, die nachfolgend möglichst unbeschädigt aus dem Kopf des Bohrers transportiert und in geeigneter Weise aufgefangen werden.

Erfindungsgemäß ist es besonders günstig, wenn der Kopf mit einem vorderen, kegelförmigen Bereich versehen ist. Dieser kann entweder rund ausgebildet sein, d.h., senkrecht zur Drehachse des Knochenspanbohrers kreisförmige Querschnitte aufweisen, als auch eine Mehrkantkontur oder Struktur haben.

Bevorzugterweise ist erfindungsgemäß die Spanausnehmung zylindrisch ausgebildet. Hierdurch wird die Förderung der Späne vereinfacht. Es ist auch möglich, die Spanausnehmung zum rückwärtigen Bereich des Kopfes, d.h., in Richtung des Schaftes geringfügig zu erweitern, um auf diese Weise ein Zusetzen der Spanausnehmung mit Spänen zu vermeiden.

Erfindungsgemäß ist es besonders vorteilhaft, wenn der Kopf einen größeren Durchmesser aufweist, als der Schaft des Knochenspanbohrers. Auf diese Weise ist ein Abtransport der Späne in Richtung des Schaftes besonders gut möglich.

Der Kopf weist angrenzend an den kegelförmigen Bereich bevorzugt einen zylindrischen Wandungsbereich auf. Dieser dient zusätzlich zur Führung während des Einsatzes des Knochenspanbohrers und kann bevorzugterweise mit einer Auffanghülse zusammenwirken, welche in günstiger Weiterbildung der Erfindung axial verschiebbar auf dem Schaft gelagert ist. Die Auffanghülse bildet dabei einen Spanraum, in welchem die Späne aufgefangen werden können. Dabei ist die Auffanghülse bevorzugterweise axial fixierbar, beispielsweise mittels einer Gewindeverschraubung.

Erfindungsgemäß ist es weiterhin besonders günstig, wenn die Spanausnehmung hinsichtlich ihrer Mittelachse in einem Winkel von 30° bis 40° zur Schaftachse geneigt ist. Bevorzugterweise beträgt der Winkel 35°. Der kegelförmige Bereich ist bevorzugterweise mit einem Kegelwinkel von im Wesentlichen 90° versehen, erfindungsgemäß sind jedoch auch kleinere oder größere Kegelwinkel günstig.

Weiterhin ist es vorteilhaft, wenn der kegelförmige Bereich an seinem vorderen Endbereich mit einer Führungsspitze versehen ist, die beispielsweise nadelartig ausgebildet ist und den Knochenspanbohrer während der Arbeit fixiert. Diese Spitze ist zudem selbstzentrierend, symmetrisch ausgebildet und verzahnt.

Der erfindungsgemäße Knochenspanbohrer wird bevorzugterweise langsam rotierend in einem Bereich von 250 bis 1000 U/min eingesetzt. Hierdurch ist ein vibrationsarmes und zentrierendes Arbeiten des Knochenspanbohrers sichergestellt.

Der erfindungsgemäße Knochenspanbohrer kann, bedingt durch seinen symmetrischen Aufbau, sowohl rechtslaufend als auch linkslaufend eingesetzt werden.

Die durch den erfindungsgemäßen Knochenspanbohrer erzeugten Späne können, wie beschrieben, durch die Auffanghülse und den durch diese gebildeten Spanraum aufgefangen werden. Es ist jedoch auch möglich, die Späne abzusaugen und in einem Knochenfilter zu sammeln, welcher beispielsweise im Bereich eines Speichelsaugers eingesetzt wird.

Im Folgenden wird die Erfindung anhand von Ausführungsbeispielen in Verbindung mit der Zeichnung beschrieben. Dabei zeigt:
- Fig. 1: eine schematische Seitenansicht eines ersten Ausführungsbeispiels,
- Fig. 2: eine Seitenansicht, analog Fig. 1, in einer um 90° gedrehten Ansicht,
- Fig. 3: eine vergrößerte Darstellung des Kopfbereiches gemäß Fig. 1,
- Fig. 4: eine vergrößerte Darstellung des Kopfbereiches gemäß Fig. 2,
- Fig. 5: eine stirnseitige Ansicht des Ausführungsbeispiels der Fig. 1-4,
- Fig. 6: eine perspektivische Ansicht eines weiteren Ausführungsbeispiels,
- Fig. 7: eine Seitenansicht des in Fig. 6 gezeigten Ausführungsbeispiels,
- Fig. 8: eine perspektivische Ansicht eines erfindungsgemäßen Knochen- spanbohrers ohne Auffanghülse,
- Fig. 9: eine Darstellung, analog Fig. 8, mit aufgesetzter Auffanghülse, und
- Fig. 10: eine Seitenansicht des Ausführungsbeispiels gemäß Fig. 9, und
- Fig. 11-13: Teil-Schnittansichten, analog Fig. 10, in unterschiedlicher Positionie- rung der Auffanghülse 9.

Bei den Ausführungsbeispielen sind gleiche Teile mit gleichen Bezugsziffern versehen.

Der erfindungsgemäße Knochenspanbohrer weist einen Kopf 1 sowie einen einstückig mit diesem verbundenen Schaft 2 auf. Der Schaft 2 ist an seinem rückwärtigen Bereich mit üblichen Einspannmitteln zum Einsetzen des Knochenspanbohrers in eine Antriebseinheit, beispielsweise ein Winkelstück, versehen.

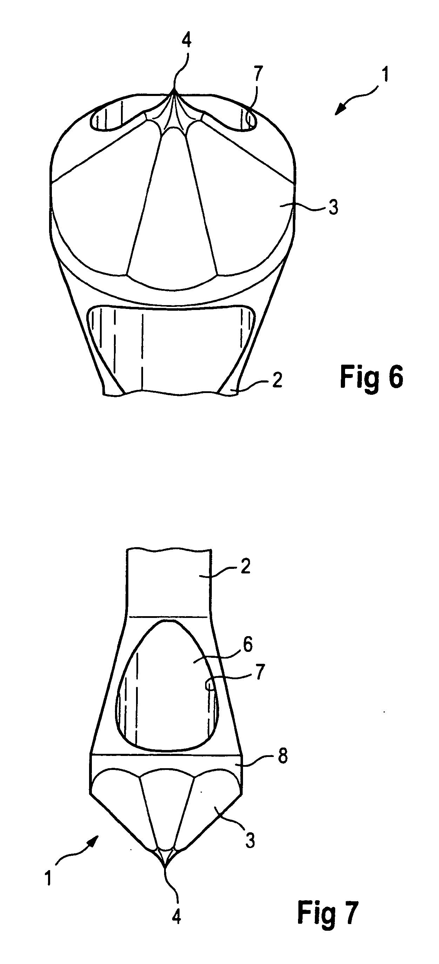

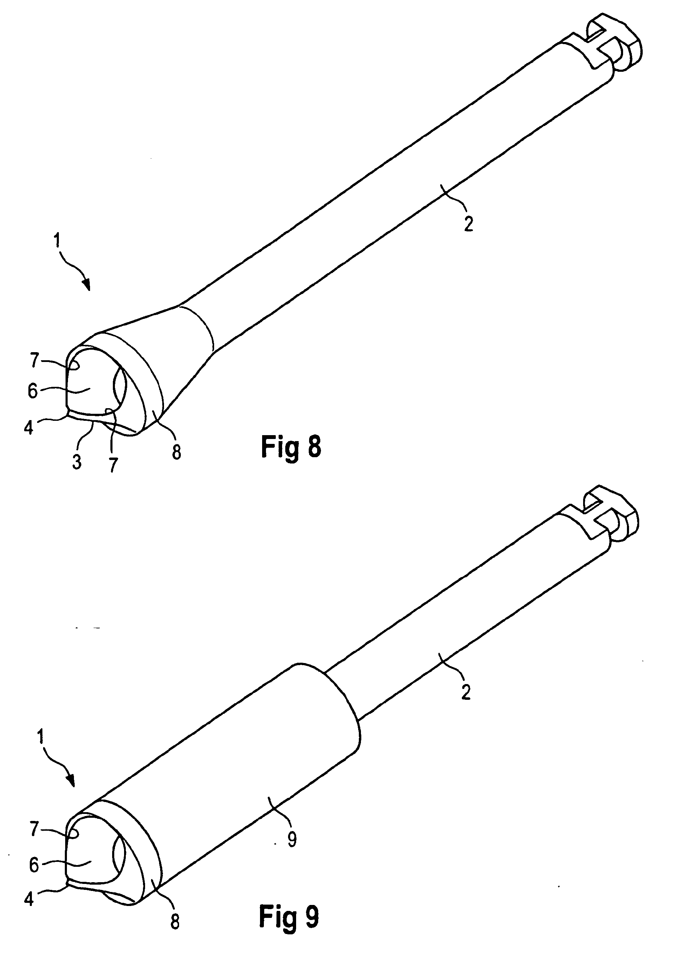

Der Kopf 1 umfasst einen vorderen, im Wesentlichen kegelförmigen Bereich 3, welcher, bezogen auf eine Schaftachse 5, in zu dieser senkrechten Schnittebenen mit einem runden Querschnitt ausgebildet ist, so wie dies beispielsweise in den Fig. 8 und 9 dargestellt ist. Der kegelförmige Bereich 3 kann jedoch auch mit einer mehrkantigen Kontur (siehe Fig. 6 und 7) versehen sein.

Der kegelförmige Bereich 3 weist an seinem vorderen Endbereich eine nadelförmige Führungsspitze 4 auf, welche zur Zentrierung des Knochenspanbohrers dient.

Der Kopf 1 ist weiterhin mit einem zylindrischen Wandungsbereich 8 versehen, welcher an den kegelförmigen Bereich angrenzt. Der dem Schaft 2 zugewandte Bereich des Kopfes 1 ist kegelförmig verjüngend ausgebildet und geht glatt in den Schaft 2 über.

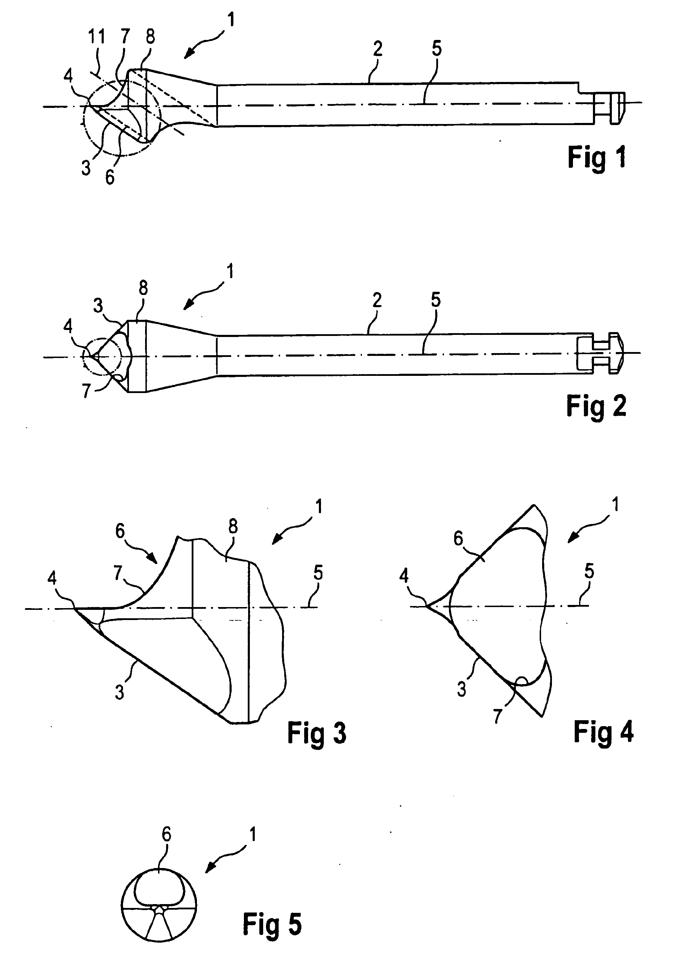

Erfindungsgemäß durchdringt den Kopf 1 eine im Wesentlichen zylindrische Spanausnehmung 6, deren Achse 11 in einem Winkel zur Schaftachse 5 geneigt ist, beispielsweise mit 35°. Die Achse 11 ist insbesondere in Fig. 1 dargestellt. Hierbei ist ersichtlich, dass sich die Spanausnehmung 6 vom kegelförmigen Bereich 3 zu dem rückwärtigen Bereich des Kopfes 1 erstreckt.

Die Spanausnehmung 6 bildet mit dem kegelförmigen Bereich 3 des Kopfes 1 eine Verschneidungskante 7, so wie dies beispielsweise in den Fig. 1 und 2 besonders deutlich dargestellt ist. Diese Kante 7 ist eine Schnittkante, mittels derer beim Vorschub des Knochenspanbohrers im Bereich des vorderen Teils des Kopfes 1 Späne erzeugt werden.

Die relativ groben Späne gelangen somit durch den Kopf 1 zu dem rückwärtigen Endbereich der Spanaushebung 6. Dort werden sie entweder abgesaugt oder in einem Spanraum 10 einer Spanhülse 9 aufgesammelt.

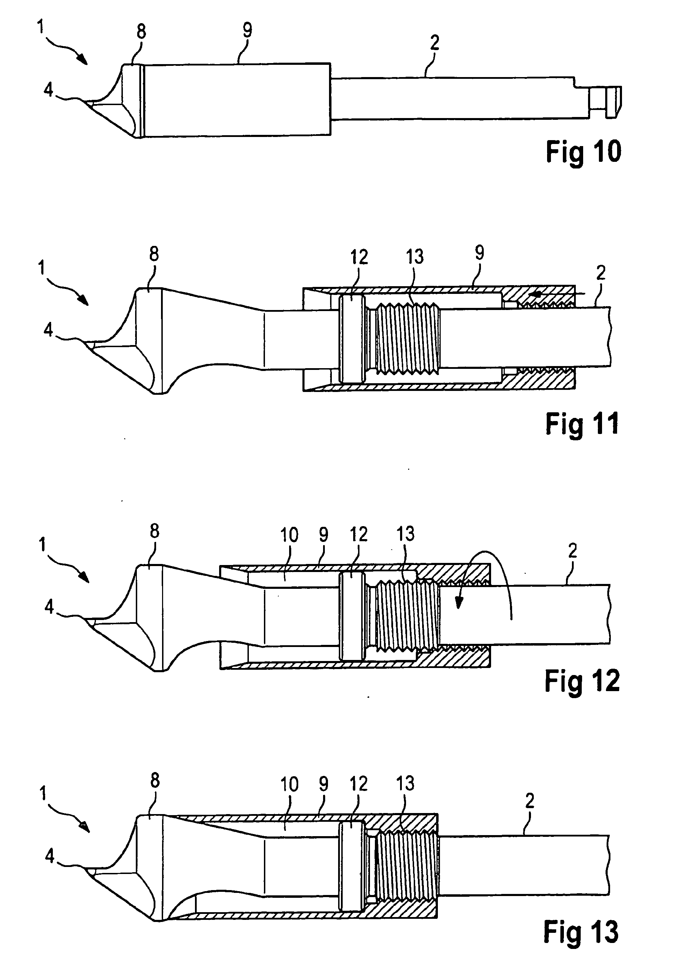

Wie insbesondere aus den Fig. 11-13 ersichtlich ist, ist die Auffanghülse 9 axial verschiebbar an dem Schaft 2 gelagert und bildet einen Spanraum 10, welcher zu seinem vorderen Endbereich durch den Kopf 1 und zu seinem hinteren Endbereich durch eine Ringwulst 12 begrenzt wird, wenn die Auffanghülse 9 sich in der Betriebsposition befindet (siehe Fig. 13). Die Auffanghülse 9 ist mit einem Innengewinde versehen, welches auf ein Gewinde 13 des Schaftes 2 aufschraubbar ist, so wie dies die Fig. 11 bis 13 darstellen.

### Bezugszeichenliste

- 1: Kopf
- 2: Schaft
- 3: Kegelförmiger Bereich
- 4: Führungsspitze
- 5: Schaftachse
- 6: Spanausnehmung
- 7: Kante
- 8: Zylindrischer Wandungsbereich
- 9: Auffanghülse
- 10: Spanraum
- 11: Achse
- 12: Ringwulst
- 13: Gewinde

## Patentansprüche

1. Knochenspanbohrer zur Gewinnung von groben Knochenspänen mit einem Kopf (1) und einem mit diesem verbundenen Schaft (2), wobei der Kopf (1) an seinem vorderen Bereich (3) im Wesentlichen kegelförmig ausgebildet ist und an seinem vorderen Endbereich mit einer Führungsspitze (4) versehen ist, wobei der Kopf (1) von einer sich diagonal und zur Schaftachse (5) geneigten Spanausnehmung (6) durchdrungen ist, deren vante (7) Verschneidungsk zumindest mit dem Kegelförmigen Bereich des Kopfes eine Schneide bildet und die sich zur Rückseite des Kopfes (1) öffnet und welche in einem Winkel zwischen 30° und 40° zur Schaftachse (5) geneigt ist, wobei der Kopf (1) mit einem zylindrischen Wandungsbereich (8), angrenzend an den kegelförmigen Bereich (3), versehen ist.

2. Knochenspanbohrer nach Anspruch 1, **dadurch gekennzeichnet, dass** der kegelförmige Bereich (3) des Kopfes (1) rund ausgebildet ist.

3. Knochenspanbohrer nach Anspruch 1, **dadurch gekennzeichnet, dass** der kegelförmige Bereich (3) des Kopfes (1) als Mehrkant ausgebildet ist.

4. Knochenspanbohrer nach einem der Ansprüche 1 bis 3, **dadurch gekennzeichnet, dass** die Spanausnehmung (6) zylindrisch ausgebildet ist.

5. Knochenspanbohrer nach einem der Ansprüche 1 bis 4, **dadurch gekennzeichnet, dass** der Durchmesser des Kopfes (1) größer als der Durchmesser des Schaftes (2) ist.

6. Knochenspanbohrer nach einem der Ansprüche 1 bis 5, **dadurch gekennzeichnet, dass** der kegelförmige Bereich (3) mit einem Kegelwinkel von im Wesentlichen 90° versehen ist.

7. Knochenspanbohrer nach einem der Ansprüche 1 bis 6, **dadurch gekennzeichnet, dass** auf dem Schaft (2) axial verschiebbar eine Auffanghülse (9) gelagert ist, welche einen Spanraum (10) bildet.

8. Knochenspanbohrer nach Anspruch 7, **dadurch gekennzeichnet, dass** die Auffanghülse (9) axial fixierbar ist.

9. Knochenspanbohrer nach Anspruch 8, **dadurch gekennzeichnet, dass** die Auffanghülse (9) zur axialen Fixierung mittels eines Gewindes mit dem Schaft verschraubbar ist.

## Claims

1. A bone graft drill for obtaining coarse bone graft chips, comprising a head (1) and a shaft (2) connected to said head, the head (1) being shaped substantially conical at a front portion (3) thereof and provided with a guiding tip (4) at a front end portion thereof, the head (1) being penetrated by a chipping recess (6) which is inclined diagonally and with respect to the shaft axis (5) and the intersection edge (7) of which constitutes at least one cutting edge with the conical portion of the head and which opens towards the rear side of the head (1) and is inclined with respect to the shaft axis (5) by an angle between 30° and 40°, the head (1) being provided with a cylindrical wall portion (8) adjacent to the conical portion (3).

2. The bone graft drill of claim 1, **characterized in that** the conical portion (3) of the head (1) is made round.

3. The bone graft drill of claim 1, **characterized in that** the conical portion (3) of the head (1) is multi-sided.

4. The bone graft drill as in any one of claims 1 to 3, **characterized in that** the chipping recess (6) is made cylindrical.

5. The bone graft drill as in any one of claims 1 to 4, **characterized in that** the diameter of the head (1) is larger than the diameter of the shaft (2).

6. The bone graft drill as in any one of claims 1 to 5, **characterized in that** the conical portion (3) is provided with a conical angle of substantially 90°.

7. The bone graft drill as in any one of claims 1 to 6, **characterized in that** a collecting sleeve (9) which forms a chipping space (10) is supported axially displaceably on the shaft (2).

8. The bone graft drill of claim 7, **characterized in that** the collecting sleeve (9) is axially fixable.

9. The bone graft drill of claim 8, **characterized in that** the collecting sleeve (9) is screwable to the shaft by a thread to be axially fixable.

## Revendications

1. Foret à fragments osseux pour l'obtention de fragments osseux grossiers, avec une tête (1) et une queue (2) assemblée à la tête, sachant que la tête (1) est réalisée essentiellement conique dans sa région avant (3) et est pourvue dans sa région terminale avant d'une pointe de guidage (4), sachant que la tête (1) est traversée par un évidement à fragments (6) s'étendant diagonalement et incliné par rapport à l'axe (5) de la queue, évidement dont l'arête (7) d'intersection avec la région conique de la tête forme au moins un tranchant et qui s'ouvre vers le côté arrière de la tête (1) et est incliné par rapport à l'axe (5) de la queue sous un angle compris entre 30° et 40°, sachant que la tête (1) est pourvue d'une région de paroi cylindrique (8), adjacente à la région conique (3).

2. Foret à fragments osseux selon la revendication 1, **caractérisé en ce que** la région conique (3) de la tête (1) est réalisée ronde.

3. Foret à fragments osseux selon la revendication 1, **caractérisé en ce que** la région conique (3) de la tête (1) est réalisée sous forme de multipans.

4. Foret à fragments osseux selon l'une des revendications 1 à 3, **caractérisé en ce que** l'évidement à fragments (6) est réalisé cylindrique.

5. Foret à fragments osseux selon l'une des revendications 1 à 4, **caractérisé en ce que** le diamètre de la tête (1) est plus grand que le diamètre de la queue (2).

6. Foret à fragments osseux selon l'une des revendications 1 à 5, **caractérisé en ce que** la région conique (3) est dotée d'un angle de cône sensiblement égal à 90°.

7. Foret à fragments osseux selon l'une des revendications 1 à 6, **caractérisé en ce qu'**un manchon collecteur (9) est monté à translation axiale sur la queue (2) et forme un logement pour fragments (10).

8. Foret à fragments osseux selon la revendication 7, **caractérisé en ce que** le manchon collecteur (9) peut être fixé en position axiale.

9. Foret à fragments osseux selon la revendication 8, **caractérisé en ce que** le manchon collecteur (9) peut, pour l'immobilisation axiale, être vissé sur la queue au moyen d'un filetage.
